# EUROPEAN PATENT SPECIFICATION

(11) **EP 3 500 225 B1**
(45) Date of publication and mention of the grant of the patent: **21.04.2021**
(21) Application number: 17758983.5
(22) Date of filing: 17.08.2017
(51) Int. Cl.: A61M 39/10, A61M 39/20, A61J 1/10, A61J 9/00, A61J 1/20

(54) **INTERNAL BOTTLE ADAPTER FOR MATERIAL TRANSFER**
INTERNER FLASCHENADAPTER ZUR MATERIALÜBERTRAGUNG
ADAPTATEUR DE BOUTEILLE INTERNE POUR TRANSFERT DE MATÉRIAU.

(30) Priority: 17.08.2016 US 201615530694; 19.08.2016 US 201615530693; 16.08.2017 US 201715678834
(43) Date of publication of application: 26.06.2019
(73) Proprietor: Taylor, Kristine Marie, Redwood City, California 94064 (US)
(72) Inventor: Taylor, Kristine Marie, Redwood City, California 94064 (US)
(74) Representative: Gulde & Partner
(86) International application number: PCT/US2017/047292
(87) International publication number: WO 2018/035293

(56) References cited:
- WO-A1-2016/035788
- US-A- 6 003 556
- US-A1- 2002 082 586
- US-A1- 2010 327 010
- US-A1- 2011 130 724
- US-B1- 6 726 672
- Anonymous: "APMF149PKG = 1/4" Male NPT x Male Luer, Straight Adapter - Bag of 10", , 8 April 2016 (2016-04-08), XP055425633, Retrieved from the Internet: URL:https://web.archive.org/web/2016040817 0951/http://www.dispensinglink.com:80/stor e/p65/APMF149PKG_=_1/4"_Male_NPT_x_Male_Lu er,_Straight_Adapter_-_Bag_of_10.html [retrieved on 2017-11-16]
- Adhesive Dispensing Ltd: "1/4" NPT to male luer plastic fitting TSD931-7C", , 4 April 2016 (2016-04-04), XP055425639, Retrieved from the Internet: URL:https://web.archive.org/web/2016040416 1631/https://www.adhesivedispensing.net/TS D931_7C_Tip_Adapter_1_4_Clr_PP_pk_5_p/tsd9 31-7c.htm [retrieved on 2017-11-16]

## Description

### BRIEF SUMMARY

Exemplary embodiments of adapters of the prior art are disclosed in US 6,003,556 A, WO 2016/035788 A1, US 2002/082586 A1, US 6,726,672 B1, US 2011/130724 A1 and US 2010/327010 A1.

The present invention is directed to subject matter as defined in the claims. The adapter of the present invention includes an adapter body with a bore through its longitudinal axis. The adapter includes a plurality ofsealing surfaces and a threaded connection nested in the adapter body. The threaded connection may be configured to mate with a CORPAK ENFIT connection or a luer lock connection.

In other embodiments, a system for accessing material in a container may include a container holding material. The adapter of the invention may have a bore and one or more sealing surfaces, where at least one sealing surface has a sealing surface diameter that is at least as large as an opening diameter of the container. The adapter has a threaded connection.

In yet other embodiments, a method of accessing a container may include inserting the adapter of the invention into a container, twisting a connector fixed to a material transfer device to engage with the adapter, sealing the container with the adapter of the invention, and adding or removing material from the container. The method may include disengaging the connector from the adapter, closing the container, opening the container, and re-engaging the connector to the adapter.

This summary is provided to introduce a selection of concepts that are further described below in the detailed description.

Additional features and advantages of embodiments of the disclosure will be set forth in the description which follows, and in part will be obvious from the description, or may be learned by the practice of such embodiments. The features and advantages of such embodiments may be realized and obtained by means of the instruments and combinations particularly pointed out in the appended claims. These and other features will become more fully apparent from the following description and appended claims, or may be learned by the practice of such embodiments as set forth hereinafter.

### BRIEF DESCRIPTION OF THE DRAWINGS

In order to describe the manner in which the above-recited and other features of the disclosure can be obtained, a more particular description will be rendered by reference to specific embodiments thereof which are illustrated in the appended drawings. For better understanding, the like elements have been designated by like reference numbers throughout the various accompanying figures. While some of the drawings may be schematic or exaggerated representations of concepts, at least some of the drawings may be drawn to scale. Understanding that the drawings depict some example embodiments, the embodiments will be described and explained with additional specificity and detail through the use of the accompanying drawings in which:
FIG. 1 is a perspective view of an adapter, according to an embodiment of the present disclosure;
FIG. 2 is a perspective view of an adapter, according to the embodiment of Figure 1;
FIG. 3 is a cross-sectional view of an adapter, according to the embodiment of Figure 1;
FIG. 4 is a cross-sectional view of an adapter, according to another embodiment of the present disclosure;
FIG. 5 is a side view of an adapter, according to another embodiment of the present disclosure;
FIG. 6 is a cut-away view of an embodiment of a system for accessing material in a container;
FIG. 7-1 and FIG. 7-2 are a cut-away views of the system of Figure 5; and
FIG. 8 is a method chart for a method of accessing a container, according to an embodiment of the present disclosure.

### DETAILED DESCRIPTION

This disclosure generally relates to devices, systems, and methods to add and remove material from a container. In particular, this disclosure discusses an adapter that is inserted into a container and connected to a material transfer device. In a hospital, home, or other patient care setting, it is critical that patients receive the correct material from the correct bottle. For example, in an enteral feeding system, it is critical that enteral food be administered, rather than another material. Similarly, for an intravenous (IV) application, it is critical that IV fluid/medication be administered, rather than another material, such as enteral food. In at least one embodiment, an adapter is provided that creates a seal against the container and fits standard enteral, IV, or other system's standard connection may help ensure that a patient receives the correct material for the right application, while maintaining ease of use for the caregiver/provider. Additionally, an adapter that is easy to install and connect may help caregivers in a fast-pace, high stress environment to quickly and efficiently administer the correct material to the correct patient.

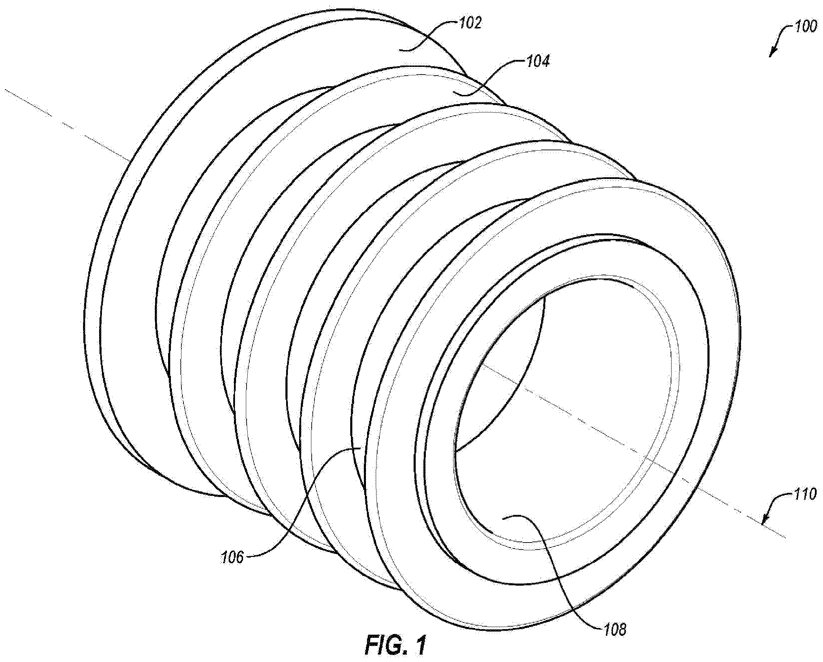

FIG. 1 is a perspective view of an adapter 100, according to an embodiment of the present disclosure. The adapter 100 includes an upper surface 102 and one or more sealing surfaces 104. The one or more sealing surfaces 104 may be used to seal an opening of a container, as described below. The adapter 100 includes an adapter body 106, the adapter body 106 having a bore 108 relative to a longitudinal axis 110 thereof. In some embodiments, the bore 108 may be positioned relative to (e.g., on or spaced away from) the longitudinal axis 110. As shown, the bore 108 may be straight. In other embodiments, the bore 108 may at least partially be non-straight (e.g., curved).

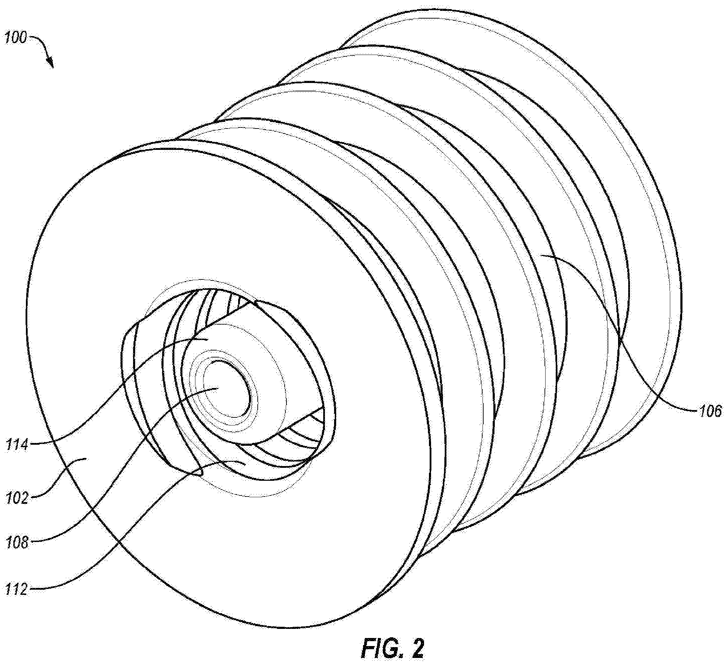

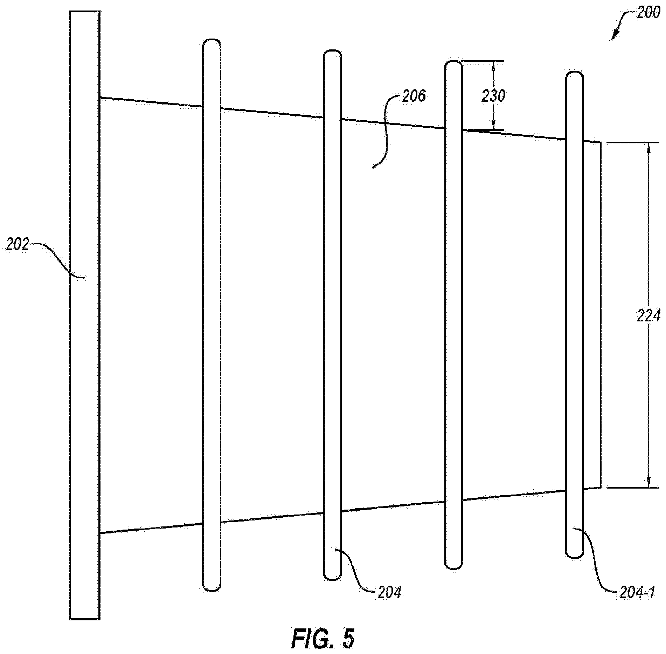

FIG. 2 is another perspective view of the embodiment of the adapter 100 of Figure 1. The adapter 100 includes a connection 112. The connection 112 may be used to connect the adapter 100 to a material transfer device (e.g., material transfer device 360 shown in Figure 5), as will be described below. The connection 112 is a threaded connection.

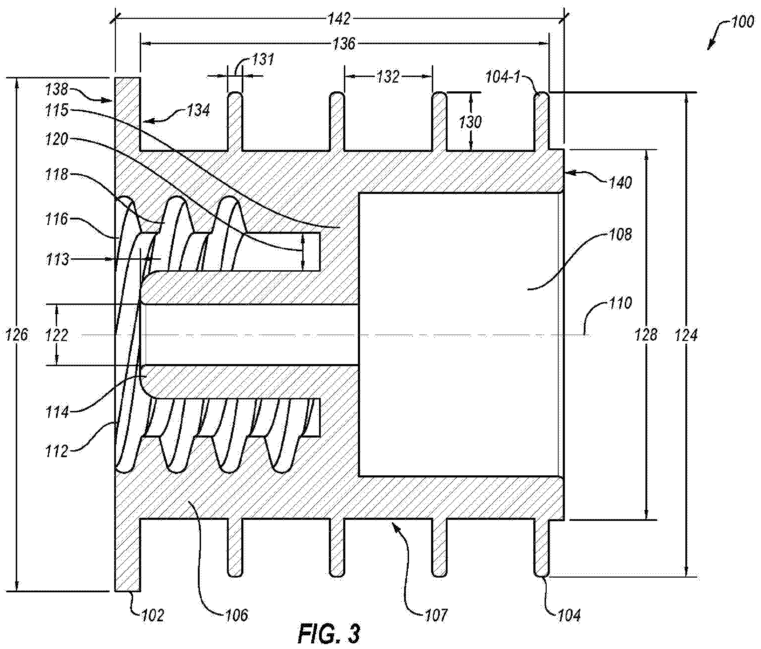

Referring now to FIG. 3, the connection 112 is nested in the adapter body 106. The connection 112 includes a port 114 through which the bore 108 extends. In some examples, the port 114 may be flush with the upper surface 102. In other examples, the port 114 may be recessed within the adapter body 106 with a recess depth 113. In some embodiments, the recess depth 113 may be in a range having an upper value, a lower value, or upper and lower values including any of 0.1 millimeters, 0.5 millimeters, 1.0 millimeters, 1.5 millimeters, 2.0 millimeters, 2.5 millimeters, 3.0 millimeters, 3.5 millimeters, 4.0 millimeters, 4.5 millimeters, 5.0 millimeters, 6.0 millimeters, 7.0 millimeters, 8.0 millimeters, 9.0 millimeters, 10 millimeters, 20 millimeters, 30 millimeters, 40 millimeters, or any value therebetween. For example, the recess depth 113 may be greater than 0.1 millimeters. In other examples, the recess depth 113 may be less than 10.0 millimeters. In yet other examples, the recess depth 113 may be in a range of 0.1 millimeters to 10.0 millimeters.

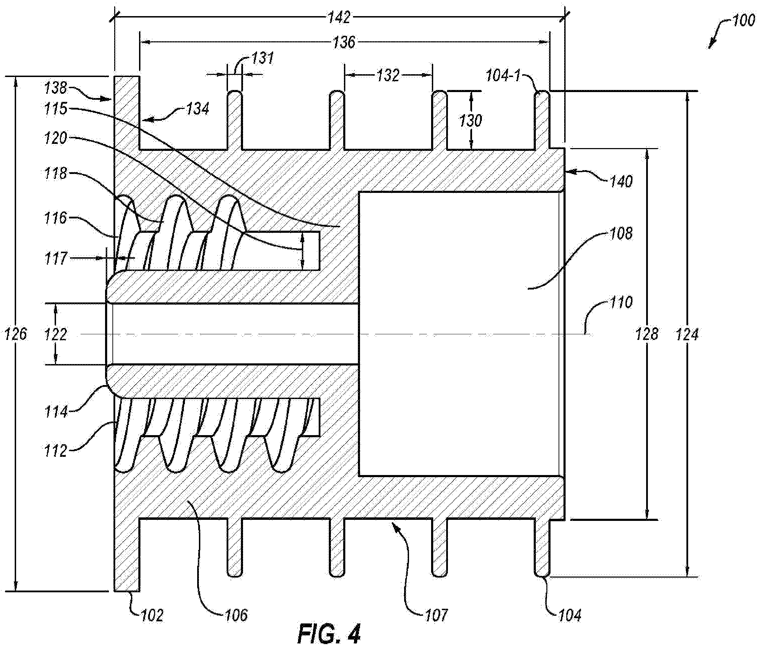

FIG. 4 represents a cross-sectional view of the embodiment of the adapter 100 of Figure 1. In some embodiments, a portion of the port 114 may extend past the upper surface 102 with a port extension height 117. In some embodiments, the port extension height 117 may be in a range having an upper value, a lower value, or upper and lower values including any of 0.1 millimeters, 0.5 millimeters, 1.0 millimeters, 1.5 millimeters, 2.0 millimeters, 2.5 millimeters, 3.0 millimeters, 3.5 millimeters, 4.0 millimeters, 4.5 millimeters, 5.0 millimeters, or any value therebetween. For example, the port extension height 117 may be greater than 0.1 millimeters. In other examples, the port extension height 117 may be less than 5.0 millimeters. In yet other examples, the port extension height 117 may be in a range of 0.1 millimeters to 5.0 millimeters.

The adapter body 106 has an outer surface 107. In some embodiments, at least one sealing surface 104 is circumscribed around the outer surface 107. In other words, at least one sealing surface 104 extends around an entirety of the circumference of the outer surface 107. The port 114 extends from a connection end 115 through an annular space 116. The annular space 116 includes threads 118, making the connection 112 a threaded connection 112. In some embodiments, the port 114 may include a valve that may restrict flow into an out of the container. Inserting a connector into the connection 112 may open the valve in the port 114, thereby allowing material to be removed from the container. In some embodiments, the valve may be a sphincter-type valve that the connector must push through to access the material in the container. In other embodiments, the valve may be a flap or bridge that may be pushed aside by the connector when the connector is installed in the connection. In still other embodiments, the valve may be a manually operated valve, such as a ball valve or a butterfly valve.

In some embodiments, the threaded connection 112 may be a standard threaded connection, such as a CORPAK ENFIT connection, a luer lock, or other standard connection Different systems may use different standard connections. For example, for enteral feeding systems, the CORPAK ENFIT connection may be a standard connection. In at least one embodiment, using a standard connection specific to enteral feeding systems may prevent accidental use of non-enteral feeding material during enteral feeding, thereby increasing patient safety. Similarly, different standard connections may be used in different contexts to prevent a health care provider from administering the wrong medication or material to a patient. For example, a luer lock connection may be used in an intravenous (IV) system, draining devices (e.g., chest tubes, urinary catheters, biliary drains), or other medical systems.

In some embodiments, the adapter 100 may include a threaded connection 112 that mates with standard connections. For example, the threaded connection may mate with the CORPAK ENFIT connection. In other examples, the threaded connection 112 may mate with a luer lock connection. In still other examples, the connection 112 may be configured to mate with any connection, including non-threaded connections.

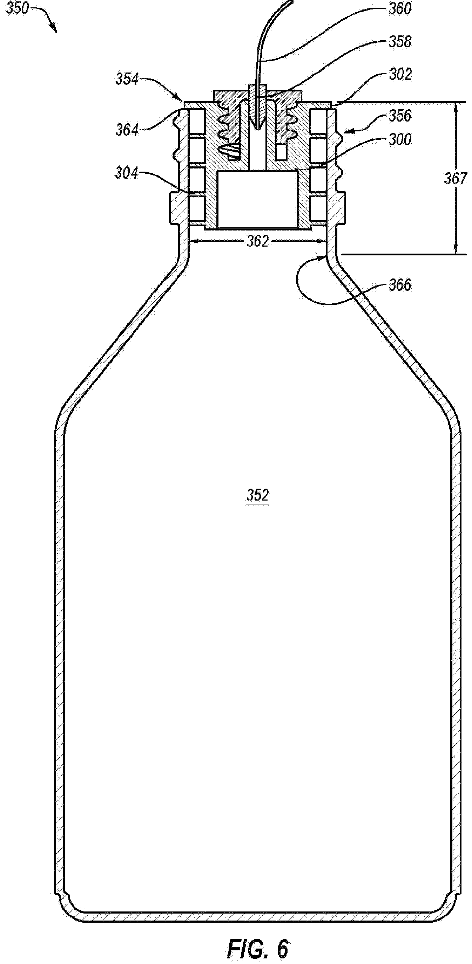

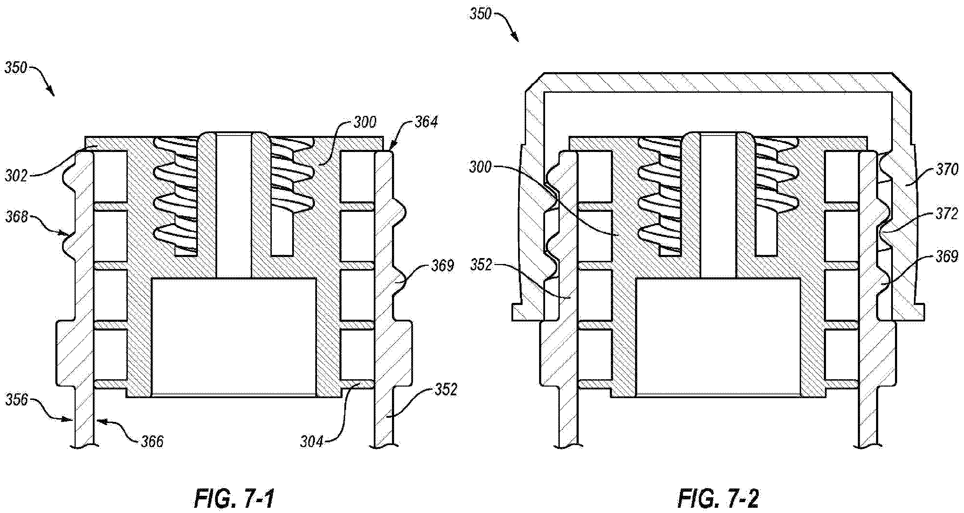

In some embodiments, the upper surface 102 may have a larger diameter than one or more of the sealing surfaces 104. For example, when the adapter 100 is inserted into a container, at least one of the sealing surfaces 104 may be configured to engage an inner surface of the container. The upper surface 102 may be sized to engage the upper edge (e.g., upper edge 364 from FIG. 6 and FIG. 7-1) of the container, thereby preventing the adapter 100 from being inserted completely into the container. In other embodiments, the upper surface 102 may have the same diameter as at least one of the sealing surfaces 104.

In some embodiments, the annular space 116 may include an annular gap 120 that is sized to receive a standard connection. In some embodiments, the annular gap 120 may be in a range having an upper value, a lower value, or upper and lower values including any of 2 millimeters, 3 millimeters, 4 millimeters, 5 millimeters, 6 millimeters, 7 millimeters, 8 millimeters, 9 millimeters, 10 millimeters, 15 millimeters, 20 millimeters, or any values therebetween. For example, the annular gap 120 may be greater than 2 millimeters. In other examples, the annular gap 120 may be less than 10 millimeters. In yet other examples, the annular gap 120 may be in a range of 2 millimeters to 10 millimeters.

In some embodiments, the port 114 includes the bore 108 with a bore diameter 122 that may be sized to receive a standard connection. In some embodiments, the bore diameter 122 may be in a range having an upper value, a lower value, or upper and lower values including any of 2 millimeters, 3 millimeters, 4 millimeters, 5 millimeters, 6 millimeters, 7 millimeters, 8 millimeters, 9 millimeters, 10 millimeters, 15 millimeters, 20 millimeters, or any values therebetween. For example, the bore diameter 122 may be greater than 2 millimeters. In other examples, the bore diameter 122 may be less than 10 millimeters. In yet other examples, the bore diameter 122 may be in a range of 2 millimeters to 10 millimeters.

Still referring to FIG. 4, in some embodiments, at least one of the sealing surfaces 104 may have a sealing surface diameter 124 that is sized to engage an inner surface of a container with a seal. In some embodiments, the sealing surface diameter 124 may be in a range having an upper value, a lower value, or upper and lower values including any of 10 millimeters, 12 millimeters, 14 millimeters, 16 millimeters, 18 millimeters, 20 millimeters, 22 millimeters, 24 millimeters, 26 millimeters, 28 millimeters, 30 millimeters, 35 millimeters, 40 millimeters, 45 millimeters, 50 millimeters, or any values therebetween. For example, the sealing surface diameter 124 may be greater than 10 millimeters. In other examples, the sealing surface diameter 124 may be less than 30 millimeters. In yet other examples, the sealing surface diameter 124 may be in a range of 10 millimeters to 30 millimeters. In some embodiments, the sealing surface diameter 124 may be the same for each sealing surface 104. In other embodiments, the sealing surface diameter 124 may be different for each sealing surface 104. In still other embodiments, the sealing surface diameter 124 may be the same for two or more sealing surfaces 104 and different for one or more sealing surface 104 on the same adapter 100.

The upper surface 102 has an upper surface diameter 126. In some embodiments, the upper surface diameter 126 may be in a range having an upper value, a lower value, or upper and lower values including any of 10 millimeters, 12 millimeters, 14 millimeters, 16 millimeters, 18 millimeters, 20 millimeters, 22 millimeters, 24 millimeters, 26 millimeters, 28 millimeters, 30 millimeters, 32 millimeters, 35 millimeters, 40 millimeters, 45 millimeters, 50 millimeters, 55 millimeters, or any values therebetween. For example, the upper surface diameter 126 may be greater than 10 millimeters. In other examples, the upper surface diameter 126 may be less than 32 millimeters. In yet other examples, the upper surface diameter 126 may be in a range of 10 millimeters to 32 millimeters. In some embodiments, the upper surface diameter 126 may be greater than the sealing surface diameter 124. In other embodiments, the upper surface diameter 126 may be approximately equal to the sealing surface diameter 124.

The adapter body 106 has an adapter body diameter 128. In some embodiments, the adapter body diameter 128 may be in a range having an upper value, a lower value, or upper and lower values including any of 8 millimeters, 10 millimeters, 12 millimeters, 14 millimeters, 16 millimeters, 18 millimeters, 20 millimeters, 22 millimeters, 24 millimeters, 26 millimeters, 28 millimeters, 30 millimeters, 35 millimeters, 40 millimeters, 45 millimeters, 50 millimeters, or any values therebetween. For example, the adapter body diameter 128 may be greater than 8 millimeters. In other examples, the adapter body diameter 128 may be less than 28 millimeters. In yet other examples, the adapter body diameter 128 may be in a range of 8 millimeters to 28 millimeters.

The difference between the sealing surface diameter 124 and the adapter body diameter 128 is the sealing height 130. In some embodiments, the sealing height 130 may be in a range having an upper value, a lower value, or upper and lower values including any of 0.1 millimeters, 0.5 millimeters, 1.0 millimeters, 1.5 millimeters, 2.0 millimeters, 2.5 millimeters, 3.0 millimeters, 3.5 millimeters, 4.0 millimeters, 4.5 millimeters, 5.0 millimeters, 10 millimeters, 15 millimeters, 20 millimeters, 25 millimeters, 30 millimeters, or any values therebetween. For example, the sealing height 130 may be greater than 0.1 millimeters. In other examples, the sealing height 130 may be less than 5.0 millimeters. In yet other examples, the sealing height 130 may be in a range of 0.0 millimeters to 5.0 millimeters. In some embodiments, the sealing height 130 may be the same for each sealing surface 104. In other embodiments, the sealing height 130 may be different for each sealing surface 104. In still other embodiments, the sealing height 130 may be the same for two or more sealing surfaces 104 and different for one or more sealing surface 104 on the same adapter 100. In some embodiments, it may be desirable to have a large sealing height 130. For example, a large sealing height 130 may accommodate a range of container opening diameters (e.g., opening diameter 362 of FIG. 6). In other embodiments, it may be desirable to have a small sealing height 130. For example, a small sealing height 130 may create a stronger seal with the container.

The sealing surface 104 has a sealing width 131. In some embodiments, the sealing width 131 may be in a range having an upper value, a lower value, or upper and lower values including any of 0.1 millimeters, 0.2 millimeters, 0.3 millimeters, 0.4 millimeters, 0.5 millimeters, 0.6 millimeters, 0.7 millimeters, 0.8 millimeters, 0.9 millimeters, 1.0 millimeters, 1.5 millimeters, 2.0 millimeters, 2.5 millimeters, 3.0 millimeters, or any values therebetween. For example, the sealing width 131 may be greater than 0.1 millimeters. In other examples, the sealing width 131 may be less than 1.0 millimeters. In yet other examples, the sealing width 131 may be in a range of 0.1 millimeters to 1.0 millimeters. In some embodiments, the sealing width 131 may be the same for each sealing surface 104. In other embodiments, the sealing width 131 may be different for each sealing surface 104. In still other embodiments, the sealing width 131 may be the same for two or more sealing surfaces 104 and different for one or more sealing surface 104 on the same adapter 100.

Still referring to FIG. 4, the ratio between the sealing height 130 and the sealing width 131 is a sealing surface aspect ratio. In some embodiments, the sealing surface aspect ratio may be in a range having an upper value, a lower value, or upper and lower values including any of 2:1, 3:1, 4:1, 5:1, 6:1, 7:1, 8:1, 9:1, 10:1, 11:1, 12:1, 13:1, 14:1, 15:1, or any values therebetween. For example, the sealing surface aspect ratio may be greater than 2:1. In other examples, the sealing surface aspect ratio may be less than 15:1. In yet other examples, the sealing surface aspect ratio may be in a range of 2:1 to 15:1. In some embodiments, the sealing surface aspect ratio may be the same for each sealing surface 104. In other embodiments, the sealing surface aspect ratio may be different for each sealing surface 104. In still other embodiments, the sealing surface aspect ratio may be the same for two or more sealing surfaces 104 and different for one or more sealing surface 104 on the same adapter 100. In some embodiments, it may be desirable to have a large sealing surface aspect ratio. For example, a large sealing surface aspect ratio may result in a flexible sealing surface 104, and may accommodate a range of container opening diameters. In other embodiments, it may be desirable to have a small sealing surface aspect ratio. For example, a small sealing surface aspect ratio may result in a stiff sealing surface 104, and create a strong seal with the container.

The space between two consecutive sealing surfaces 104 has a sealing surface spacing 132. In some embodiments, the sealing surface spacing 132 may be in a range having an upper value, a lower value, or upper and lower values including any of 1.0 millimeters, 1.5 millimeters, 2.0 millimeters, 2.5 millimeters, 3.0 millimeters, 3.5 millimeters, 4.0 millimeters, 4.5 millimeters, 5.0 millimeters, 6.0 millimeters, 7.0 millimeters, 8.0 millimeters, 9.0 millimeters, 10.0 millimeters, or any values therebetween. For example, the sealing surface spacing 132 may be greater than 1.0 millimeters. In other examples, the sealing surface spacing 132 may be less than 5.0 millimeters. In yet other examples, the sealing surface spacing 132 may be in a range of 1.0 millimeters to 10.0 millimeters.

In some embodiments, the sealing surface spacing 132 may be the same between each sealing surface 104. In other embodiments, the sealing surface spacing 132 may be different between sealing surfaces 104. In still other embodiments, the sealing surface spacing 132 may be the same for two or more sealing surfaces 104 and different for one or more sealing surface 104 on the same adapter 100. In some embodiments, it may be desirable to have a small sealing surface spacing 132. For example, a small sealing surface spacing 132 may result in the sealing surfaces stacking to create a strong seal. In other embodiments, it may be desirable to have a large sealing surface spacing 132. For example, a large sealing surface spacing 132 may result in a plurality of individual seals against the container, thereby creating redundant seals.

The ratio between the sealing surface spacing 132 and the sealing height 130 is a sealing spacing aspect ratio. In some embodiments, the sealing spacing aspect ratio may be in a range having an upper value, a lower value, or upper and lower values including any of 1:5, 1:4, 1:3, 1:2, 1:1, 2:1, 3:1, 3:1, 5:1, or any values therebetween. For example, the sealing spacing aspect ratio may be greater than 1:5. In other examples, the sealing spacing aspect ratio may be less than 5:1. In yet other examples, the sealing spacing aspect ratio may be in a range of 1:5 to 5:1. In some embodiments, it may be desirable to have a small sealing spacing aspect ratio. For example, a small sealing spacing aspect ratio may result in the sealing surfaces stacking to create a strong seal. In other embodiments, it may be desirable to have a large sealing spacing aspect ratio. For example, a large sealing spacing aspect ratio may result in a plurality of individual seals against the container, thereby creating redundant seals.

In some embodiments, and as shown in FIG. 4, the adapter 100 may include four sealing surfaces 104. In other embodiments, the adapter 100 may include 1, 2, 3, 4, 5, or 6 sealing surfaces 104. In some embodiments, the upper surface 102 may be a sealing surface.

The space between a lower face 134 of the upper surface 102 and the last sealing surface 104-1 is the sealing length 136. In some embodiments, the sealing length 136 may be in a range having an upper value, a lower value, or upper and lower values including any of 4 millimeters, 6 millimeters, 8 millimeters, 10 millimeters, 12 millimeters, 14 millimeters, 16 millimeters, 18 millimeters, 20 millimeters, 22 millimeters, 24 millimeters, 26 millimeters, 28 millimeters, 30 millimeters, 35 millimeters, 40 millimeters, or any values therebetween. For example, the sealing length 136 may be greater than 4 millimeters. In other examples, the sealing length 136 may be less than 30 millimeters. In yet other examples, the sealing length 136 may be in a range of 4 millimeters to 30 millimeters. In some embodiments, it may be desirable to have a long sealing length 136. For example, a long sealing length 136 may create a stronger seal against the container. In other embodiments, it may be desirable to have a short sealing length 136. For example, a short sealing length 136 may match the length of a short container neck (e.g., neck 556 of FIG. 6), thereby preventing the trapping of material between the outer surface 107 and the container.

The ratio between the adapter body diameter 128 and the sealing length 136 is an adapter body aspect ratio. In some embodiments, the adapter body aspect ratio may be in a range having an upper value, a lower value, or upper and lower values including any of 1:5, 1:4, 1:3, 1:2, 1:1, 2:1, 3:1, 3:1, 5:1, or any values therebetween. For example, the adapter body aspect ratio may be greater than 1:5. In other examples, the adapter body aspect ratio may be less than 5:1. In yet other examples, the adapter body aspect ratio may be in a range of 1:5 to 5:1.

The ratio between the sealing surface diameter 124 and the sealing length 136 is a sealing surface diameter aspect ratio. In some embodiments, the sealing surface diameter aspect ratio may be in a range having an upper value, a lower value, or upper and lower values including any of 1:5, 1:4, 1:3, 1:2, 1:1, 2:1, 3:1, 4:1, 5:1, or any values therebetween. For example, the sealing surface diameter aspect ratio may be greater than 1:5. In other examples, the sealing surface diameter aspect ratio may be less than 5:1. In yet other examples, the sealing surface diameter aspect ratio may be in a range of 1:5 to 5:1.

The length from an upper face 138 of the upper surface 102 and the end 140 of the adapter 100 is the adapter length 142. In some embodiments, the adapter length 142 may be in a range having an upper value, a lower value, or upper and lower values including any of 6 millimeters, 8 millimeters, 10 millimeters, 12 millimeters, 14 millimeters, 16 millimeters, 18 millimeters, 20 millimeters, 22 millimeters, 24 millimeters, 26 millimeters, 28 millimeters, 30 millimeters, 32 millimeters, 35 millimeters, 40 millimeters, or any values therebetween. For example, the adapter length 142 may be greater than 6 millimeters. In other examples, the adapter length 142 may be less than 32 millimeters. In yet other examples, the adapter length 142 may be in a range of 6 millimeters to 32 millimeters.

The ratio between the sealing surface diameter 124 and the adapter length 142 is an adapter length aspect ratio. In some embodiments, the adapter length aspect ratio may be in a range having an upper value, a lower value, or upper and lower values including any of 1:6, 1:5, 1:4, 1:3, 1:2, 1:1, 2:1, 3:1, 3:1, 5:1, 6:1, or any values therebetween. For example, the adapter length aspect ratio may be greater than 1:5. In other examples, the adapter length aspect ratio may be less than 5:1. In yet other examples, the adapter length aspect ratio may be in a range of 1:5 to 5:1.

In some embodiments, the last sealing surface 104-1 may be the end 140 of the adapter 100. In other embodiments, the adapter body 106 may extend past the last sealing surface 104-1.

In some embodiments, the adapter 100 may be made from plastic or rubber. In some embodiments, the adapter 100 may be manufactured in a sterile manufacturing plant and shipped in sterile packaging. In some embodiments, the adapter 100 may be made from a sterilizable material. For example, the adapter 100 may be made from polyethylene, other plastics, rubber, silicone, glass, other sterilizable materials, or combinations thereof.

In some embodiments, the adapter 100 may include a directional air valve. The directional air valve may be configured to allow air to enter the container but prevent material from exiting the container. In some embodiments, the directional air valve may be located on the upper surface next to the connection. In other embodiments, the directional air valve may be located on the upper surface radially outward from the adapter body. In some embodiments, a series of directional air valves may be located on the upper surface and sealing surfaces to pass air into the container. For example, the directional air valve may be a flap that covers a hole in the body 106, the flap being on the side of the adapter 100 that includes the material. In other examples, the directional air valve may be two opposing flaps that overlap over a hole in the body 106. A trigger pressure will push the opposing flaps into the container and release a quantity of air into the container.

FIG. 5 is another embodiment of an adapter 200. The adapter 200 may be similar to the adapter 100 shown in FIGS. 1-4 in at least one aspect. Like numerals represent like elements and the disclosure of FIGS. 1-4 is hereby incorporated into the description of the adapter 200. For example, the adapter 200 may include an adapter body 206, an upper surface 202, one or more sealing surfaces 204, and a connector (not shown). As shown in FIG. 5, the adapter body 206 may include a taper between the upper surface 202 and a last sealing surface 204-1. As shown in Figure 3, sides of the adapter body 106 may be parallel.

In some embodiments, each sealing surface 204 may have the same sealing surface diameter 224. In other embodiments, such as the embodiment shown in FIG. 5, each sealing surface 204 may have different sealing surface diameters 224. In some embodiments, the sealing surface diameter 224 may be the same for two or more sealing surfaces 204 and different for one or more sealing surface 204 on the same adapter 200. In some embodiments, each sealing surface 204 may have the same sealing height 230. In other embodiments, each sealing surface 204 may have different sealing heights 230. In some embodiments, the sealing height 230 may be the same for two or more sealing surfaces 204 and different for one or more sealing surface 204 on the same adapter 200.

FIG. 6 represents a system 350 for accessing material in a container. The system 350 includes a container 352. In some embodiments, the container 352 may be a bottle. In other embodiments, the container 352 may be another type of container such as a bag or other container. The container 352 may contain a material. In some embodiments, the material may be food for an enteral feeding system. In other embodiments, the material may be a beneficial agent (e.g., medicine). In still other embodiments, the material may be saline solution. In yet other embodiments, the material may be any material stored in a container. In further embodiments, the material may be collected in a container. In at least one embodiment, the container may be sterile to store and/or collect material.

The container 352 includes a container opening 354. In some embodiments, material may be removed from the container 352 through the container opening 354. In some embodiments, the container opening 354 may be located at a neck 356. The neck 356 may restrict the width of the container 352 such that the container opening 354 is narrower than the container 352. In other embodiments, the neck 356 of the container 352 may not restrict the width of the container 352 such that the container opening 354 has the same width as the container 352. In other words, the container 352 and the neck 356 may be parallel similar to a test tube.

Removing material from the container in one or more of a controlled, sanitary, and safe manner may be critical in certain industries. For example, simply pouring material from the container opening 354 increases the risk of spillage. Controlling the material removed from the container 352 may be accomplished by using an adapter 300 that includes a threaded connection nested in an adapter body (e.g., adapter designed to mate with a connector 358 of a material transfer device 360. In some embodiments, the adapter 300 may be inserted into the container opening 354. At least one sealing surface 304 of the adapter 300 may be sized to fit within the container opening 354. In some embodiments, the at least one sealing surface 304 may have a sealing surface diameter (e.g., sealing surface diameter 124, as shown in relation to FIG. 4) that is approximately the same as a container opening diameter 362. In some embodiments, the sealing surface diameter is at least as large as or greater than the container opening diameter 362.

In some embodiments, an upper surface 302 of the adapter 300 may have a greater upper surface diameter (e.g., upper surface diameter 126, as shown in relation to FIG. 4) than the container opening diameter 362. In this manner, when the adapter 300 is inserted into the container opening 354, the upper surface 302 may engage an upper edge 364 of the container 352, thereby prohibiting further insertion into the container 352. In other embodiments, the upper surface 302 may have an upper surface diameter that is the same as or less than the container opening diameter 362. In some embodiments, the upper surface 302 may be inserted into the container opening 354, and may act as a sealing surface 304.

In some embodiments, one or more sealing surfaces 304 may contact an inner surface 366 of the neck 356. The contact between the one or more sealing surfaces 304 and the inner surface 366 may create a seal between the adapter 300 and the container 352. Thus, in some embodiments, when a material transfer device 360 is connected to the adapter 300, the only path through which material may enter or exit the container 352 may be through the material transfer device 360.

In some embodiments, a connection port (e.g., port 114 from FIG. 4) may include a valve that may restrict flow of material out of the container 352 except when the connector 358 of the material transfer device 360 is connected to the adapter. A port valve may help prevent accidental spills and contamination of the material.

In some embodiments, the adapter 300 may include a last sealing surface 304 (e.g., last sealing surface 104-1 from FIG. 4) that has a sealing surface diameter that is wider than the other sealing surfaces 304. The last sealing surface 304 may engage the inner surface 366 of the container 352 as the inner surface 366 flares outward, thereby increasing the strength and integrity of the seal. In some embodiments, the adapter 300 may include a plurality of sealing surfaces that flare outward in relation to the inner surface 366.

The adapter 300 has a removal force. The removal force is the force required to remove the adapter from the container 352. In some embodiments, the removal force may be in a range having an upper value, a lower value, or upper and lower values including any of 2.0 Newtons (N), 3.0 N, 4.0 N, 5.0 N, 7.5 N, 10 N, 20 N, 30 N, 40 N, 50 N, 75 N, 100 N, 125 N, 150 N, 175 N, 200 N, or any values therebetween. For example, the removal force may be greater than 2.0 N. In other examples, the removal force may be less than 200 N. In yet other examples, the removal force may be in a range of 2.0 N to 200 N. In some embodiments, it may be desirable to have a large removal force. For example, a large removal force means that the adapter 300 is hard to remove, thereby decreasing the chance of an accidental removal due to squeezing the container 352 or flipping the container 352 upside down. In other embodiments, it may be desirable to have a small removal force. For example, a small removal force may result in an adapter 300 that is easy to remove.

In some embodiments, the adapter 300 may include a directional air valve, which may allow air into the container 352, but prevent material from escaping the container 352 through the directional valve. For example, a pinhole opening may be used that is sized to allow the passage of air while preventing passage of the material. In such an example, a removable cover may be used to maintain sterility of the material. In another example, a one way valve may be used.

The neck 356 has a restriction length 367, which is the length of the container 352 that the neck 356 is restricted to the opening diameter 362 or close to the opening diameter 362. In some embodiments, the restriction length 367 may be the same as, or approximately the same as, the sealing length (e.g., the sealing length 136, as shown in reference to FIG. 4). In other embodiments, the restriction length 367 may be greater than the sealing length. In still other embodiments, the restriction length 367 may be less than the sealing length. In some embodiments, the apparatus body between two sealing surfaces 304 may include one or more gaps in fluid communication with a bore through the apparatus body. The one or more gaps may allow material to flow from the container and into the bore. For example, if the restriction length 367 is less than the sealing length, material may be trapped between the adapter and the inner surface 366 of the container 352. One or more gaps in the adapter body may allow the material to flow into the bore and prevent waste.

In some embodiments, the connector 358 may complementarily fit the connection of the adapter 300. For example, the connector 358 may be the male end of a CORPAK ENFIT enteral feeding system connection, and the connection in the adapter 300 may be the female end of the CORPAK ENFIT enteral feeding system connection. In other examples, the connector 358 may be male end of a luer lock, and the connection of the adapter 300 may be the female end of a luer lock. Although typically material is removed from a bottle using a female adapter, in some embodiments, the connection in the adapter 300 may be the male end of the CORPAK ENFIT enteral feeding system connection. In other embodiments, the connection in the adapter 300 may be the male end of a luer lock.

In some embodiments, the material transfer device 360 may be manually operated, such as a syringe. In other embodiments, the material transfer device 360 may be a pump, such as an electric pump. In still other embodiments, the material transfer device 360 may be a gravity-fed tube. In some embodiments, the material transfer device 360 may remove material from the container 352. In other embodiments, the material transfer device 360 may add material to the container 352. In still other embodiments, the material transfer device 360 may add and/or remove material to the container 352.

In some embodiments, in an installed configuration, the adapter 300 may be completely internal to the container 352. For example, the entire adapter 300 may be located entirely within the container 352 such that the upper surface 302 of the adapter 300 is below an upper edge 364 of the container 352. In other embodiments, in the installed configuration, the adapter 300 may be partially internal to the container 352. For example, in the installed configuration, all of the adapter 300 except the upper surface 302 (e.g., including the upper face 138 and the lower face 134 of FIG. 4) may be internal to the container. In other words, the lower face 134 may abut the upper edge 364 of the container 352. In other examples, all of the adapter 300 except the upper surface 302 (e.g., including the upper face 138 and the lower face 134) and one, two, three, four, or five sealing surfaces 304 may be internal to the container 352.

In some embodiments, all of the sealing surfaces 304 may be internal to the container 352. In other embodiments, not all of the sealing surfaces 304 may be internal to the container 352. For example, in an adapter 300 with five sealing surfaces 304, two sealing surfaces 304 may be internal to the container 352. In other examples, in an adapter 300 with three sealing surfaces 304, two sealing surfaces 304 may be internal to the container 352. In yet other examples, in an adapter with four sealing surfaces, one sealing surface 304 may be internal to the container 352.

In some embodiments, the adapter 300 may be tapered as shown in FIG. 5. In this embodiment, an adapter 300 may be inserted into a container 352 until at least one of the sealing surfaces 304 engages the inner wall 366. Because of the taper, this may result in one or more sealing surface 304 being inside the container 352 but not contacting the inner wall 366, one or more sealing surface 304 engaging the inner wall 366, and one or more sealing surface 304 being outside the container 352.

FIG. 7-1 is a partial cutaway view of the neck 356 of the container 352 of the system 350. In some embodiments, the adapter 300 may be inserted into the neck 356, and the sealing surfaces 304 may contact the inner surface 366 of the neck 356. The upper surface 302 may overlap an upper edge 364 of the neck 356. In some embodiments, the upper surface 302 may partially overlap the upper edge 364. In other embodiments, the upper surface 302 may completely overlap the upper edge 364. The upper surface 302 may extend to the neck outer surface 368. In some embodiments, the upper surface 302 may extend past the neck outer surface 368. The neck 356 may include container threads 369.

Referring now to FIG. 7-2, the system 350 may include a container lid 370. The container lid 370 may have lid threads 372 complementary to the container threads 369. In some embodiments, the container lid 370 may completely enclose the adapter 300 in a closed configuration. For example, the container lid 370 may surround the entirety of the upper surface 302 of the adapter 300. In some embodiments, the container lid 370 may seal the container 352 when enclosing the adapter 300 in the closed configuration.

In some embodiments, the container lid 370 may be a screw-on lid. In other embodiments, the container lid 370 may be a safety-lid (e.g., child locked). In still other embodiments, the container lid 370 may be a press-fit lid. In still other embodiments, the container lid 370 may close with a latch or other locking mechanism.

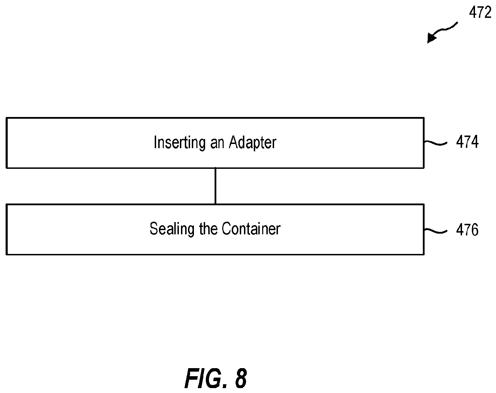

FIG. 8 is an embodiment of a method 472 of installing an adapter according to at least one embodiment of the present disclosure. The method 472 may include inserting 474 an adapter into the neck or opening of a container. In some embodiments, a container lid may be removed from the container prior to inserting 474 the adapter into the neck or opening of the container. The adapter may be inserted 474 into the neck or opening of the container such that it is completely or substantially completely enclosed in the container. The adapter may include one or more sealing surfaces and a threaded connection. In some embodiments, inserting 474 the adapter may occur during assembly of the container. In other embodiments, inserting 474 of the adapter may occur after filling the container with material but prior to shipping the bottle. In still other embodiments, inserting 474 the adapter may occur in a professional setting, such as at a hospital. In yet other embodiments, inserting 474 the adapter may occur in a user's home.

The method 472 may include the act of sealing 476 the container by engaging at least one sealing surface of the adapter against an inner surface of the container. In some embodiments, the adapter may completely seal 476 the container, such that no material may be removed prior to connection of a material transfer device. In other embodiments, the adapter may seal 476 the container except for a bore (e.g., bore 108 of FIG. 4) through the adapter. In some embodiments, sealing 476 the container may include creating a water-tight seal. In other embodiments, sealing 476 the container may include creating an air-tight seal. In still other embodiments, sealing 476 the container may create a seal strong enough to withstand rotating the container upside down such that the entire weight of the material inside the container is sealed by the adapter.

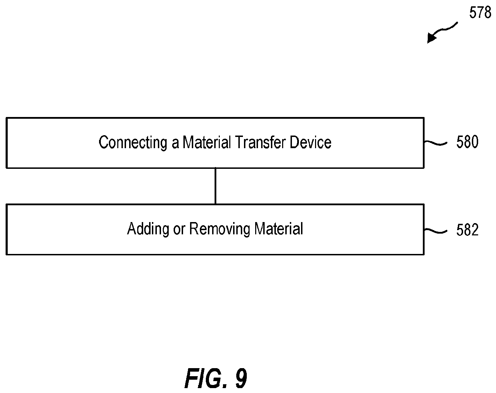

FIG. 9 is an embodiment of a method 578 of accessing a container according to at least one embodiment of the present disclosure. The method 578 may include connecting 580 a connector fixed to a material transfer device to a connection in an adapter. Connecting 580 may include twisting the connector in the connection to connect a CORPAK ENFIT connection, luer lock connection, or other connection. In some embodiments, connecting 580 the connector may seal the adapter. In some embodiments, the method 578 may include the act of adding or removing 582 material from the container. Adding or removing 582 material from the container may include manually adding or removing 582, using a pump to add or remove 582 material, or using a gravity- feed to add or remove 582 material.

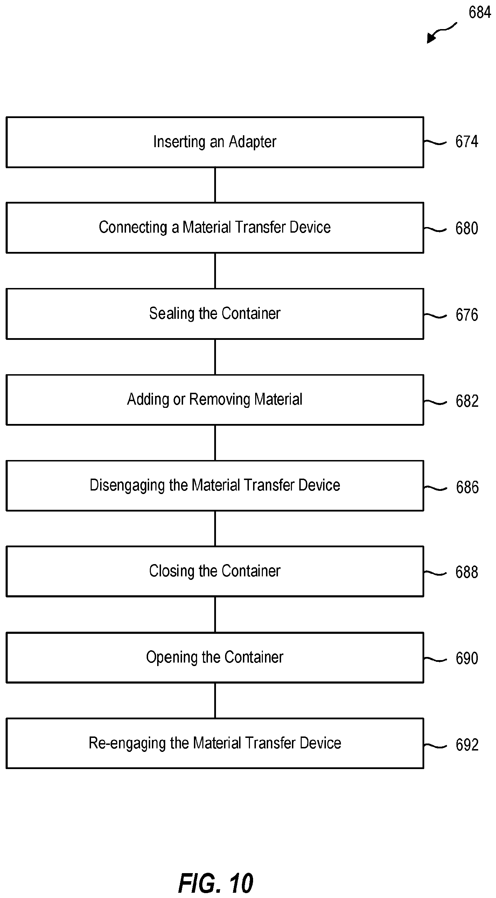

FIG. 10 is an embodiment of a method 684 of accessing a container according to at least one embodiment of the present disclosure. Similar to method 472 discussed in reference to FIG. 8, the method 684 may include inserting 674 an adapter into the container and sealing 676 the container. The method 684 may include some or all of the acts discussed in reference to FIG. 8, which is incorporated herein.

In some embodiments, and similar to method 578 discussed in reference to FIG. 9, the method 684 may include connecting 680 a material transfer device and adding or removing 682 material from the container. The method 684 may include some or all of the acts discussed in reference to FIG. 9, which is incorporated herein.

In some embodiments, the method 684 may include venting the container using a directional air valve in the adapter. As material is removed, the directional air valve may allow air into the container while flow of the material out of the container through the directional air valve.

In some embodiments, the method 684 may include disengaging 686 the connector of the material transfer device from the adapter by twisting the connector in an opposite direction used in the connecting 680 and removing the material transfer device. The method 684 may also include closing 688 the container with a container lid. Closing 688 the container may include installing a container lid in a closed configuration with the adapter still inserted in the neck or opening of the container. Closing 688 the container may create a water and/or air tight seal between the container lid and the adapter, thereby preventing any material from entering or exiting the container.

After the container is closed 688, the container may be stored or transported for use in a different location. Leaving the adapter in the container after it is closed may save some of the time, hassle, and expense often expended in changing out adapters.

In some embodiments, the method 684 may include opening 690 the container. The connector of the material transfer device may then be re-engaged 690 with the threaded connection of the adapter without removing or reinserting the adapter. Reusing the same container, with the same adapter, may save significant time, hassle, and expense in finding and replacing adapters that cannot be closed within the container. This may also help prevent spills caused by over-handling the container. Additionally, storing the adapter in the container may help ensure that the correct adapter is used when material is removed from the bottle, because there will not be an opportunity to select the incorrect adapter.

The method 684 may include any single act or combination of acts referenced in FIG. 10. For example, the method 684 may include inserting 674 an adapter, sealing 676 the container, and closing 688 the container. In other examples, the method 684 may include connecting 680 the material transfer device, and disengaging 686 the material transfer device.

One or more specific embodiments of the present disclosure are described herein. These described embodiments are examples of the presently disclosed techniques.

The articles "a," "an," and "the" are intended to mean that there are one or more of the elements in the preceding descriptions. The terms "comprising," "including," and "having" are intended to be inclusive and mean that there may be additional elements other than the listed elements. Additionally, it should be understood that references to "one embodiment" or "an embodiment" of the present disclosure are not intended to be interpreted as excluding the existence of additional embodiments that also incorporate the recited features. For example, any element described in relation to an embodiment herein may be combinable with any element of any other embodiment described herein. Numbers, percentages, ratios, or other values stated herein are intended to include that value, and also other values that are "about" or "approximately" the stated value, as would be appreciated by one of ordinary skill in the art encompassed by embodiments of the present disclosure. A stated value should therefore be interpreted broadly enough to encompass values that are at least close enough to the stated value to perform a desired function or achieve a desired result. The stated values include at least the variation to be expected in a suitable manufacturing or production process, and may include values that are within 5%, within 1%, within 0.1%, or within 0.01% of a stated value.

A person having ordinary skill in the art should realize in view of the present disclosure that equivalent constructions do not depart from the scope of the present disclosure, and that various changes, substitutions, and alterations may be made to embodiments disclosed herein without departing from the scope of the present disclosure. Equivalent constructions, including functional "means-plus-function" clauses are intended to cover the structures described herein as performing the recited function, including both structural equivalents that operate in the same manner, and equivalent structures that provide the same function. It is the express intention of the applicant not to invoke means-plus-function or other functional claiming for any claim except for those in which the words 'means for' appear together with an associated function. Each addition, deletion, and modification to the embodiments that falls within the meaning and scope of the claims is to be embraced by the claims.

The terms "approximately," "about," and "substantially" as used herein represent an amount close to the stated amount that still performs a desired function or achieves a desired result. For example, the terms "approximately," "about," and "substantially" may refer to an amount that is within less than 5% of, within less than 1% of, within less than 0.1% of, and within less than 0.01% of a stated amount. Further, it should be understood that any directions or reference frames in the preceding description are merely relative directions or movements. For example, any references to "up" and "down" or "above" or "below" are merely descriptive of the relative position or movement of the related elements.

The present disclosure may be embodied in other specific forms. The invention is defined by the scope of the appended claims.

## Claims

1. An adapter, comprising:
an adapter body (106) having a bore (108) relative to a longitudinal axis (110) thereof, the adapter body (106) including an upper face (138) at an end of the adapter body (106);
a plurality of sealing surfaces (104) circumscribed around an outer surface (107) of the adapter body (106) and having a sealing surface diameter (124) that is greater than an adapter body diameter (128) of the adapter body (106);
an upper surface (102) at the upper face (138);
**characterized in that**
the adapter comprises a threaded connection (112) nested in the adapter body (106), including:
a connection end (115) between the upper face (138) and the end of the adapter body (106);
a port (114) extending from the connection end (115) toward the upper face (138), the bore (108) extending through the port (114);
an annular space (116), the port (114) extending through the annular space (116) toward the upper face (138); and
threads (118) in the annular space (116).

2. The adapter of claim 1, wherein the upper surface (102) including an upper surface diameter (126), wherein the upper surface diameter (126) is greater than the sealing surface diameter (124).

3. The adapter of claim 1 or claim 2, wherein at least two sealing surfaces (104) of the plurality of sealing surfaces (104) have different sealing surface diameters (124).

4. The adapter of any of claims 1-3, wherein the threaded connection (112) mates with a luer lock connection.

5. The adapter of any of claims 1-3, wherein the threaded connection (112) mates with a standard threaded connection.

6. A system for accessing material in a container, comprising:
a container (352) having an opening (354) with an opening diameter, the container (352) including material; and
the adapter of any of claims 1-5.

7. The system of claim 6, further comprising a material transfer device (360) having a connector complementary to the threaded connection (112).

8. The system of claim 7, wherein the material transfer device (360) is gravity fed.

9. The system of claim 7, wherein the material transfer device (360) is a syringe.

10. The system of claim 7, wherein the material transfer device (360) includes an electric pump.

11. The system of any of claims 6-10, wherein the system is an enteral feed system.

12. The system of any of claims 6-11, further comprising a container lid (370), wherein the container lid (370) encloses the adapter in an installed configuration.

13. A method of installing an adapter into a container, comprising:
inserting the adapter of any of claims 1-12 into the container of any of claims 6-12; and
sealing the container by engaging at least one of the one or more sealing surfaces against an inner surface of the container.

14. The method of claim 13, further comprising closing the container with a container lid over the adapter.

15. The method of claim 14, further comprising opening the container by removing the container lid over the adapter and engaging a connector of a material transfer device with the threaded connection.

## Patentansprüche

1. Adapter, umfassend:
einen Adapterkörper (106), der eine Bohrung (108) relativ zu einer Längsachse (110) davon aufweist, wobei der Adapterkörper (106) an einem Ende des Adapterkörpers (106) eine Oberseite (138) beinhaltet;
eine Mehrzahl von Dichtflächen (104), die eine Außenfläche (107) des Adapterkörpers (106) umschließen und einen Dichtflächendurchmesser (124) aufweisen, der größer ist als ein Adapterkörperdurchmesser (128) des Adapterkörpers (106);
eine obere Fläche (102) an der Oberseite (138);
**dadurch gekennzeichnet, dass** der Adapter eine Gewindeverbindung (112) umfasst, die in den Adapterkörper (106) eingebettet ist, die Folgendes beinhaltet:
ein Verbindungsende (115) zwischen der Oberseite (138) und dem Ende des Adapterkörpers (106);
einen Anschluss (114), der sich von dem Verbindungsende (115) in Richtung der Oberseite (138) erstreckt, wobei sich die Bohrung (108) durch den Anschluss (114) erstreckt;
einen Ringraum (116), wobei sich der Anschluss (114) durch den Ringraum (116) in Richtung der Oberseite (138) erstreckt; und
Gewinde (118) in dem Ringraum (116).

2. Adapter nach Anspruch 1, wobei die obere Fläche (102) einen Durchmesser (126) der oberen Fläche beinhaltet, wobei der Durchmesser (126) der oberen Fläche größer ist als der Dichtflächendurchmesser (124).

3. Adapter nach Anspruch 1 oder 2, wobei mindestens zwei Dichtflächen (104) der Mehrzahl von Dichtflächen (104) unterschiedliche Dichtflächendurchmesser (124) aufweisen.

4. Adapter nach einem der Ansprüche 1 bis 3, wobei die Gewindeverbindung (112) mit einer Luer-Lock-Verbindung zusammenpasst.

5. Adapter nach einem der Ansprüche 1 bis 3, wobei die Gewindeverbindung (112) mit einer Standardgewindeverbindung zusammenpasst.

6. System für den Zugriff auf Material in einem Behälter, umfassend:
einen Behälter (352), der eine Öffnung (354) mit einem Öffnungsdurchmesser aufweist,
wobei der Behälter (352) Material beinhaltet; und
den Adapter nach einem der Ansprüche 1 bis 5.

7. System nach Anspruch 6, ferner umfassend eine Materialübertragungsvorrichtung (360), die einen Verbinder aufweist, der komplementär zu der Gewindeverbindung (112) ist.

8. System nach Anspruch 7, wobei die Materialübertragungsvorrichtung (360) schwerkraftgespeist ist.

9. System nach Anspruch 7, wobei die Materialübertragungsvorrichtung (360) eine Spritze ist.

10. System nach Anspruch 7, wobei die Materialübertragungsvorrichtung (360) eine elektrische Pumpe beinhaltet.

11. System nach einem der Ansprüche 6 bis 10, wobei das System ein enterales Nahrungssystem ist.

12. System nach einem der Ansprüche 6 bis 11, ferner umfassend einen Behälterdeckel (370), wobei der Behälterdeckel (370) den Adapter in einer installierten Konfiguration umschließt.

13. Verfahren zur Installation eines Adapters in einem Behälter, umfassend:
Einsetzen des Adapters nach einem der Ansprüche 1 bis 12 in den Behälter nach einem der Ansprüche 6 bis 12; und
Abdichten des Behälters durch Ineingriffbringen mindestens einer der einen oder mehreren Dichtflächen mit einer Innenfläche des Behälters.

14. Verfahren nach Anspruch 13, ferner umfassend das Schließen des Behälters mit einem Behälterdeckel über dem Adapter.

15. Verfahren nach Anspruch 14, ferner umfassend das Öffnen des Behälters durch Entfernen des Behälterdeckels über dem Adapter und Ineingriffbringen eines Verbinders einer Materialübertragungsvorrichtung mit der Gewindeverbindung.

## Revendications

1. Adaptateur, comprenant :
un corps d'adaptateur (106) ayant un alésage (108) relativement à un axe longitudinal (110) de celui-ci, le corps d'adaptateur (106) incluant une face supérieure (138) au niveau d'une extrémité du corps d'adaptateur (106) ;
une pluralité de surfaces d'étanchéité (104) circonscrites autour d'une surface externe (107) du corps d'adaptateur (106) et ayant un diamètre de surface d'étanchéité (124) qui est supérieur à un diamètre de corps d'adaptateur (128) du corps d'adaptateur (106) ;
une surface supérieure (102) au niveau de la face supérieure (138) ;
**caractérisé en ce que**
l'adaptateur comprend une connexion filetée (112) emboîtée dans le corps d'adaptateur (106), incluant :
une extrémité de connexion (115) entre la face supérieure (138) et l'extrémité du corps d'adaptateur (106) ;
un port (114) s'étendant à partir de l'extrémité de connexion (115) vers la face supérieure (138), l'alésage (108) s'étendant au travers du port (114) ;
un espace annulaire (116), le port (114) s'étendant au travers de l'espace annulaire (116) vers la face supérieure (138) ; et
des filetages (118) dans l'espace annulaire (116).

2. Adaptateur selon la revendication 1, dans lequel la surface supérieure (102) inclut un diamètre de surface supérieure (126), le diamètre de surface supérieure (126) étant supérieur au diamètre de surface d'étanchéité (124).

3. Adaptateur selon la revendication 1 ou la revendication 2, dans lequel au moins deux surfaces d'étanchéité (104) parmi la pluralité de surfaces d'étanchéité (104) ont des diamètres de surface d'étanchéité différents (124).

4. Adaptateur selon l'une quelconque des revendications 1 à 3, dans lequel la connexion filetée (112) s'accouple avec une connexion Luer Lock.

5. Adaptateur selon l'une quelconque des revendications 1 à 3, dans lequel la connexion filetée (112) s'accouple avec une connexion filetée standard.

6. Système pour accéder à un matériau dans un contenant, comprenant :
un contenant (352) ayant une ouverture (354) munie d'un diamètre d'ouverture, le contenant (352) incluant un matériau ; et
l'adaptateur selon l'une quelconque des revendications 1 à 5.

7. Système selon la revendication 6, comprenant en outre un dispositif de transfert de matériau (360) ayant un connecteur complémentaire de la connexion filetée (112).

8. Système selon la revendication 7, dans lequel le dispositif de transfert de matériau (360) est alimenté par gravité.

9. Système selon la revendication 7, dans lequel le dispositif de transfert de matériau (360) est une seringue.

10. Système selon la revendication 7, dans lequel le dispositif de transfert de matériau (360) inclut une pompe électrique.

11. Système selon l'une quelconque des revendications 6 à 10, dans lequel le système est un système d'alimentation entérale.

12. Système selon l'une quelconque des revendications 6 à 11, comprenant en outre un couvercle de contenant (370), dans lequel le couvercle de contenant (370) entoure l'adaptateur dans une configuration installée.

13. Procédé d'installation d'un adaptateur dans un contenant, comprenant :
l'insertion de l'adaptateur selon l'une quelconque des revendications 1 à 12 dans le contenant selon l'une quelconque des revendications 6 à 12 ; et
l'étanchéification du contenant par engagement d'au moins une des une ou plusieurs surfaces d'étanchéité contre une surface interne du contenant.

14. Procédé selon la revendication 13, comprenant en outre la fermeture du contenant avec un couvercle de contenant au-dessus de l'adaptateur.

15. Procédé selon la revendication 14, comprenant en outre l'ouverture du contenant par retrait du couvercle de contenant au-dessus de l'adaptateur et engagement d'un connecteur d'un dispositif de transfert de matériau avec la connexion filetée.
